(19) Europäisches Patentamt
European Patent Office
Office européen des brevets

(11) Veröffentlichungsnummer: **0 546 395 B1**

(12) # EUROPÄISCHE PATENTSCHRIFT

(45) Veröffentlichungstag der Patentschrift: **25.10.95**

(51) Int. Cl.[6]: **C07D 309/08**, C07C 67/475

(21) Anmeldenummer: **92120257.8**

(22) Anmeldetag: **27.11.92**

(54) **Verfahren zur Herstellung von Carbonsäureestern.**

(30) Priorität: **13.12.91 DE 4141223**

(43) Veröffentlichungstag der Anmeldung:
**16.06.93 Patentblatt 93/24**

(45) Bekanntmachung des Hinweises auf die Patenterteilung:
**25.10.95 Patentblatt 95/43**

(84) Benannte Vertragsstaaten:
**BE CH DE FR GB IT LI NL**

(56) Entgegenhaltungen:

**TETRAHEDRON LETTERS, Bd. 27, Nr. 20, 1986, Oxford, GB, Seiten 2283 - 2286 A. KRIEF, ET AL.: 'Novel synthesis of methyl caronate'**

(73) Patentinhaber: **BASF Aktiengesellschaft**
**Carl-Bosch-Strasse 38**
**D-67063 Ludwigshafen (DE)**

(72) Erfinder: **Fischer, Rolf, Dr.**
**Bergstrasse 98**
**W-6900 Heidelberg (DE)**
Erfinder: **Goetz, Norbert, Dr.**
**Schoefferstrasse 25**
**W-6520 Worms 1 (DE)**
Erfinder: **Kuekenhoehner, Thomas, Dr.**
**Forststrasse 104**
**W-6737 Boehl-Iggelheim (DE)**
Erfinder: **Schnurr, Werner, Dr.**
**Im Eulengeschrei 3**
**W-6719 Herxheim (DE)**

**EP 0 546 395 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Monocarbonsäureestern durch Umsetzung von geminalen Dicarbonsäureestern in Gegenwart von Metalloxiden bei erhöhten Temperaturen.

Es ist bekannt, geminale Dicarbonsäureester in dreistufiger Reaktion durch alkalische Verseifung zur entsprechenden geminalen Dicarbonsäure, thermische Abspaltung einer Carboxylgruppe und Veresterung der verbleibenden Carboxylgruppe - in Monocarbonsäureester umzuwandeln (JP-A-62/201 843; Chem. Abstr.: Vol. 109, 14925). Weiterhin ist bekannt, aus geminalen Dicarbonsäureestern durch Erhitzen mit Gemischen aus Mineralsäuren und Carbonsäuren entsprechende Monocabonsäuren zu erhalten (Arch. Pharm. 319, Seiten 29 bis 37 (1986)), die anschließend zu den gewünschten Monocarbonsäureestern verestert werden müssen. Ferner ist aus Tetrahedron Lett. 27, Seiten 2283 bis 2286 (1986) bekannt, geminale Dicarbonsäureester einstufig durch Erhitzen in polaren Lösungsmitteln wie Dimethylsulfoxid oder Dimethylformamid in Gegenwart von Alkalichloriden oder Cyaniden in Monocarbonsäureester umzuwandeln.

Die beiden ersten Methoden zur Herstellung von Carbonsäureestern aus geminalen Dicarbonsäureestern besitzen den Nachteil, daß mehrere Reaktionsstufen erforderlich sind und daß im ersten Fall Salzanfall auftritt. Für die dritte, einstufige Synthese sind sehr lange Reaktionszeiten erforderlich.

Der verliegenden Erfindung lag daher die Aufgabe zugrunde, den zuvor genannten Nachteilen abzuhelfen.

Demgemäß wurde ein neues und verbessertes Verfahren zur Herstellung von Monocarbonsäureestern der allgemeinen Formel I entwickelt,

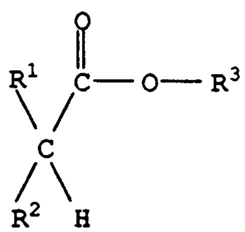

I,

in der

| | |
|---|---|
| $R^1$, $R^2$ | Wasserstoff, $C_1$- bis $C_{12}$-Alkyl, $C_3$- bis $C_8$-Cycloalkyl, Acyl, Aryl oder $C_7$- bis $C_{20}$-Aralkyl oder gemeinsam $-(CH_2)_n-X-(CH_2)_m-$, |
| X | Methylen, Sauerstoff, Schwefel, NH oder $NR^3$ |
| $R^3$ | $C_1$- bis $C_{12}$-Alkyl und |
| n, m | 0 bis 8 |

bedeuten, welches dadurch gekennzeichnet ist, daß man geminale Dicarbonsäureester der allgemeinen Formel II

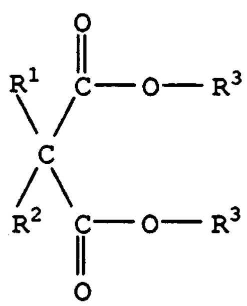

II,

in der $R^1$ bis $R^3$ die obengenannten Bedeutungen haben, in Gegenwart von sauren Katalysatoren bei Temperaturen von 150 bis 400 °C umsetzt.

Das erfindungsgemäße Verfahren läßt sich wie folgt durchführen:

Die gemialen Dicarbonsäureester II werden bei erhöhten Temperaturen durch Umsetzung an sauren Katalysatoren in die Monoester I umgewandelt.

Die Umsetzung kann man diskontinuierlich oder kontinuierlich bei Temperaturen von 150 bis 400 °C, vorzugsweise von 200 bis 400 °C, insbesondere von 250 bis 350 °C durchführen. Der Druck bei der Reaktion ist unkritisch, vorteilhaft wählt man Drücke von 1 bis 100 bar, insbesondere von 1 bis 10 bar. Die

Umsetzung der geminalen Dicarbonsäurediester II zu Monocarbonsäureestern I kann in der Flüssigphase oder bevorzugt in der Gasphase erfolgen. Man arbeitet dabei vorteilhaft mit einer Katalysatorbelastung von 0,1 bis 10 g, insbesondere 0,1 bis 5 g Dicarbonsäureester II pro g Katalysator und Stunde.

Die erfindungsgemäße Umsetzung kann in Abwesenheit von Lösungsmitteln durchgeführt werden. Es kann jedoch vorteilhaft sein, in Anwesenheit von Lösungsmitteln zu arbeiten. Als Lösungsmittel können beispielsweise Ether wie Diethylether, Tetrahydrofuran und Dioxan, Aromaten wie Benzol, Toluol und Xylole, chlorierte Kohlenwasserstoffe wie Chloroform und Methylenchlorid, Alkohole z.B. $C_1$- bis $C_8$-Alkohole, bevorzugt $C_1$- bis $C_5$-Alkohole wie Methanol, Ethanol, n-Propanol, i-Propanol, Butanole und Pentanole verwendet werden. Als besonders vorteilhaft haben sich Alkohole erwiesen. Die Menge an Lösungsmittel beträgt, bezogen auf den eingesetzten geminalen Dicarbonsäurediester II, 5 bis 90 Gew.-%.

Die Umsetzung kann diskontinuierlich oder kontinuierlich als Festbettreaktion mit Festbettkatalysatoren, beispielsweise in Sumpf- oder Rieselfahrweise in der Flüssigphase oder in der Gasphase, z. B. im Wirbelbett oder aber mit in der Flüssigphase suspendierten Festbettkatalysatoren durchgeführt werden.

Die Umsetzung der geminalen Dicarbonsäureester II in der Flüssigphase wird beispielsweise so durchgeführt, daß man ein Gemisch aus II und gegebenenfalls einem Lösungsmittel in Gegenwart eines suspendierten Festbettkatalysators auf die gewünschte Reaktionstemperatur erhitzt. Nach Ablauf der Reaktion wird das Reaktionsgemisch abgekühlt und der Katalysator, z.B. durch Filtration oder Neutralisation, entfernt.

Das Reaktionsgemisch kann anschließend zur Gewinnung der gewünschten Monocarbonsäureester I fraktionierend destilliert werden.

Nach einer bevorzugten Ausführungsform des erfindungsgemäßen Verfahrens in der Gasphase an Wirbelbettkatalysatoren und in Gegenwart eines Alkohols kann z.B. folgendermaßen verfahren werden: Ein Gemisch aus II und dem jeweiligen Alkohol wird verdampft und dann, gegebenenfalls zusammen mit einem Inertgas wie Stickstoff, Kohlendioxid oder Argon, bei der gewünschten Reaktionstemperatur gasförmig über einen fest angeordneten Katalysator geleitet, wobei der Katalysator vorteilhaft in auf- und abwirbelnder Bewegung gehalten wird. Der Reaktionsaustrag wird mittels geeigneter Kühlvorrichtung kondensiert und anschließend durch fraktionierende Destillation aufgearbeitet. Der gewünschte Monocarbonsäureester I wird abgetrennt. Nicht umgesetzter Dicarbonsäureester II kann gegebenenfalls in die Reaktion zurückgeführt werden.

Grundsätzlich sind verschiedenste saure Katalysatoren wie Mineralsäuren, Sulfonsäuren, Carbonsäuren und Lewissäuren geeignet.

Als saure Katalysatoren eignen sich besonders Metalloxide der Elemente der ersten bis fünften Hauptgruppe und der ersten bis achten Nebengruppe sowie Oxide der Lanthaniden. Beispiele für derartige Metalloxide sind Bortrioxid, Aluminiumoxid, Siliziumdioxid, Titandioxid, Zinkoxid, Nioboxid, Vanadinpentoxid, Molybdänoxid, Ceroxid und Wolframoxid, bevorzugt Aluminiumoxid, Siliziumdioxid, Titandioxid, Zirkondioxid, Bortrioxid, Vanadinpentoxid, Molybdänoxid, Wolframoxid Chromoxid oder deren Gemische.

Als saure Katalysatoren eignen sich ferner saure Phosphate, saure Ionenaustauscher, Silikate und Zeolithe wie beispielsweise Zeolithe der Mordenit-Gruppe, X-, Y- oder L-Zeolithe, wie Mordenit, Erionit und Fonjasit, bevorzugt Zeolithe mit Pentasilstruktur wie ZSM-5-, ZSM-11- und ZBM-10-Zeolithe, besonders bevorzugt ZSM-5- und ZSM-11-Zeolithe.

Als saure Katalysatoren eignen sich weiterhin Heteropolysäuren wie $H_3[PW_{12}O_{40}]$, $H_3[PMo_{12}O_{40}]$ oder $H_4[SiW_{12}O_{40}]$, bevorzugt $H_3[PW_{12}O_{40}]$ und $H_3[PMo_{12}O_{40}]$, besonders bevorzugt $H_3[PW_{12}O_{40}]$.

Das Zwischenglied X, die Substituenten $R^1$, $R^2$ und $R^3$ und die Indizes n und m in den Verbindungen I und II haben folgende Bedeutungen:

$R^1$, $R^2$

- unabhängig voneinander
- Wasserstoff,
- $C_1$- bis $C_{12}$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt $C_1$- bis $C_8$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt $C_1$- bis $C_4$-Alkyl wie Methyl, Ethyl, n-Propyl, iso-Propyl, n-Butyl, iso-Butyl, sec.-Butyl und tert.-Butyl,
- $C_3$- bis $C_8$-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl,
- Acyl wie Acetyl,

- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- $C_7$- bis $C_{20}$-Aralkyl, bevorzugt $C_7$- bis $C_{12}$-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- $-(CH_2)_n-X-(CH_2)_m-$,

X

- Methylen ($-CH_2-$),
- Sauerstoff,
- Schwefel,
- NH,
- $NR^3$,

$R^3$

- $C_1$- bis $C_{12}$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt $C_1$- bis $C_8$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt $C_1$- bis $C_4$-Alkyl wie Methyl, Ethyl, n-Propyl, iso-Propyl, n-Butyl, iso-Butyl, sec.-Butyl und tert.-Butyl,

n, m

- jeweils eine ganze Zahl von 0 bis 8 wie 0, 1, 2, 3, 4, 5, 6, 7 und 8, bevorzugt jeweils eine ganze Zahl von 1 bis 4 wie 1, 2, 3 und 4.

Geeignete geminale Dicarbonsäureester der Formel II sind in der $R^1$ und $R^2$ gemeinsam $-(CH_2)_n-X-(CH_2)_m-$, X Methylen, Sauerstoff, Schwefel, NH oder $NR^3$; $R^3$ $C_1$- bis $C_{12}$-Alkyl und n,m 0 bis 8 in den Formel I und II bedeuten.

Geeignete geminale Dicarbonsäureester der Formel II sind z.B. in 2-Stellung substituierte Malonsäurediester wie 2-Methylmalonsäuredimethylester, 2-Ethylmalonsäurediethylester, 2-Acetylmalonsäuredimethylester, 2-Phenylmalonsäurediethylester, 2.2-Dimethylmalonsäuredimethylester, 2.2-Diacetylmalonsäuredipropylester, 2-Benzylmalonsäuredimethylester, 1.1-Cyclopropandicarbonsäuredimethylester, 1.1-Cyclohexandicarbonsäurediethylester, Tetrahydropyran-4.4-dicarbonsäuredimethylester, Tetrahydrothiopyran-4.4-dicarbonsäuredimethylester, 1.1-Cyclopentandicarbonsäuredimethylester.

Die für die erfindungsgemäße Umsetzung benötigten Dicarbonsäureester II sind z.B. durch Mono- oder Bisalkylierung oder Acylierung von Malonsäurediestern zugänglich.

Die nach dem erfindungsgemäßen Verfahren erhältlichen Monocarbonsäureester stellen wertvolle, vielseitig für organische Synthese verwendbare Zwischenprodukte dar.

## Beispiele

### Beispiele 1 bis 6

In den Beispielen 1 bis 5 wurde Tetrahydropyran-4.4-dicarbonsäuredimethylester in der Gasphase an oxidischen Festbettkatalysatoren - mit oder ohne Lösungsmittel - zu Tetrahydropyran-4-carbonsäuremethylester umgesetzt:

In Beispiel 1 wurden pro Stunde 8 g Tetrahydropyran-4.4-dicarbonsäuredimethylester bei verschiedenen Temperaturen auf 5 g $Al_2O_3$ gepumpt, das sich in einer Edelstahl-Reaktorwendel befand. Der Reaktor wurde in einem Heißluftofen auf die gewünschte Temperatur aufgeheizt. Der gasförmige Austrag wurde kondensiert und gaschromatographisch analysiert. In der Tabelle ist die gaschromatographisch ermittelte Zusammensetzung des Reaktionsaustrags (ohne Lösungsmittel) nach der jeweiligen Reaktionszeit angegeben.

In den Beispielen 2 bis 6 wurden pro Stunde Lösungen von ca. 5 g Tetrahydropyran-4.4-dicarbonsäuredimethylester in jeweils ca. 5 g Lösungsmittel zugefahren. Der in Beispiel 3 bei 270 °C über einen Zeitraum von 6 Stunden erhaltene flüssige Austrag (51,6 g) wurde fraktionierend destilliert. Hierbei wurden 18,7 g Tetrahydropyran-4-carbonsäuremethylester (82 %, bezogen auf 32,2 g eingesetzten Dicarbonsäureester) erhalten.

Beispiel 7

Pro Stunde wurde eine Lösung von 4,4 g 2.2-Dimethylmalonsäuredimethylester, in 4,4 g Methanol, bei 275 °C über 5 g Aluminiumoxid geleitet. Der innerhalb von 6 Stunden Reaktionszeit erhaltende Reaktionsaustrag (42,5 g) ergab nach Destillation ein Gemisch aus 10,7 g 2.2-Dimethylmalonsäuremethylester (64 %, bezogen auf eingesetzten Diester) und 8 g Methanol.

**Tabelle: Tetrahydropyrancarbonsäureester (2) durch Gasphasen-Spaltung von Tetrahydropyrandicarbonsäureester (1)**

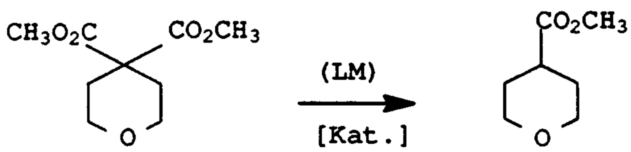


(1) (2)

| Bsp. | Katalysator (Festbett) | Lösungsmittel Art | Gew.% | Temp. [°C] | Reakt. Zeit [h] | GC-Analysen (Fl%) (1) | (2) | Summe der Nebenpro. |
|---|---|---|---|---|---|---|---|---|
| 1 | $Al_2O_3$ | -- | -- | 270 | 3 | 81 | 11 | 8 |
| | | | | 300 | 2 | 88 | 7 | 5 |
| | | | | 350 | 2 | 61 | 22 | 17 |
| 2 | | THF | 50 | 270 | 2 | 76 | 13 | 11 |
| | | | | 300 | 2 | 85 | 9 | 6 |
| | | | | 330 | 1 | 73 | 17 | 10 |
| 3 | | $CH_3OH$ | 50 | 270 | 4 | 6 | 86 | 8 |
| | | | | 300 | 2 | 5 | 45 | 50 |
| 4 | $SiO_2$ | $CH_3OH$ | 50 | 270 | 2 | 94 | 4 | 2 |
| | | | | 300 | 2 | 88 | 11 | 1 |
| | | | | 350 | 1,5 | 74 | 25 | 1 |
| | | | | 400 | 1 | 50 | 48 | 2 |
| | | | | 450 | 1 | 34 | 60 | 6 |
| | $TiO_2$ | $CH_3OH$ | 50 | 270 | 2 | 67 | 31 | 2 |

| Bsp. | Katalysator (Festbett) | Lösungsmittel Art | Gew.% | Temp. [°C] | Reakt. Zeit [h] | GC-Analysen (Fl%) (1) | (2) | Summe der Nebenpro. |
|---|---|---|---|---|---|---|---|---|
| 5 | | | | 300 | 2 | 46 | 53 | 1 |
| | | | | 350 | 2 | 20 | 76 | 4 |
| | | | | 400 | 1,5 | 18 | 62 | 20 |
| 6 | ZnO | $CH_3OH$ | 50 | 270 | 1,5 | 93 | 6 | 1 |
| | | | | 300 | 2 | 90 | 9 | 1 |
| | | | | 350 | 2 | 82 | 16 | 2 |
| | | | | 400 | 1,5 | 69 | 15 | 16 |

Beispiel 8

Pro Stunde wurde eine Lösung von 4,5 g 1.1-Cyclohexandicarbonsäuredimethylester in 4,5 g Methanol bei 275 °C über 5 g Aluminiumoxid gefahren. Der innerhalb von 6 Stunden erhaltene Reaktionsaustrag (36,5 g) ergab nach Destillation 10,1 g Cyclohexancarbonsäuremethylester (53 %, bezogen auf eingesetzten Dicarbonsäureester) und 7,7 g Ausgangsprodukt.

Beispiel 9

Eine Lösung von 4,5 g 1.1-Cyclopropandicarbonsäurediethylester in 4,5 g Ethanol wurde bei 300 °C über 5 g Aluminiumoxid geleitet. Der Reaktionsaustrag bestand laut GC-Analyse (Flächen-%) zu 58 % aus Ausgangsprodukt, zu 35 % aus Cyclopropancarbonsäureethylester und zu 7 % aus Nebenkomponenten.

## Patentansprüche

**1.** Verfahren zur Herstellung von Monocarbonsäureestern der allgemeinen Formel I

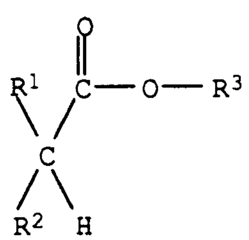

I,

in der

| | |
|---|---|
| $R^1$, $R^2$ | Wasserstoff, $C_1$- bis $C_{12}$-Alkyl, $C_3$- bis $C_8$-Cycloalkyl, Acyl, Aryl oder $C_7$- bis $C_{20}$-Aralkyl oder gemeinsam $-(CH_2)_n-X-(CH_2)_m-$, |
| X | Methylen, Sauerstoff, Schwefel, NH oder $NR^3$ |
| $R^3$ | $C_1$- bis $C_{12}$-Alkyl und |
| n, m | 0 bis 8 |

bedeuten, dadurch gekennzeichnet, daß man geminale Dicarbonsäureester der allgemeinen Formel II

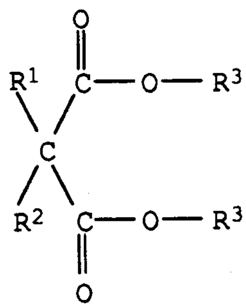

II,

in der $R^1$ bis $R^3$ die obengenannten Bedeutungen haben, in Gegenwart von sauren Katalysatoren bei Temperaturen von 150 bis 400 °C umsetzt.

2. Verfahren nach Anspruch 1, dadurch gekennzeichnet, daß man als saure Katalysatoren Metalloxide der Elemente der ersten bis fünften Hauptgruppe und/oder der ersten bis achten Nebengruppe und/oder der Lanthanidengruppe des Periodensystems der Elemente und/oder Zeolithe und/oder Heteropolysäuren verwendet.

3. Verfahren nach Anspruch 1, dadurch gekennzeichnet, daß man als saure Katalysatoren Aluminiumoxid, Siliciumdioxid, Titandioxid, Zirkondioxid, Vanadinpentoxid, Bortrioxid und/oder Oxide des Chroms, Molybdäns und/oder Wolframs verwendet.

4. Verfahren nach Anspruch 1, dadurch gekennzeichnet, daß man als Katalysatoren Zeolithe oder Heteropolysäuren verwendet.

5. Verfahren nach Anspruch 1, dadurch gekennzeichnet, daß in der Gasphase gearbeitet wird.

6. Verfahren nach Anspruch 1, dadurch gekennzeichnet, daß $R^1$ und $R^2$ gemeinsam $-(CH_2)_n-X-(CH_2)_m-$, X Methylen, Sauerstoff, Schwefel, NH oder $NR^3$; $R^3$ $C_1$- bis $C_{12}$-Alkyl und n,m 0 bis 8 in den Formel I und II bedeuten.

7. Verfahren nach Anspruch 1, dadurch gekennzeichent, daß $R^1$ und $R^2$ gemeinsame $-(CH_2)_2-O-(CH_2)_2-$ in den Formeln I und II bedeutet.

## Claims

1. A process for preparing monocarboxylic esters of the general formula I

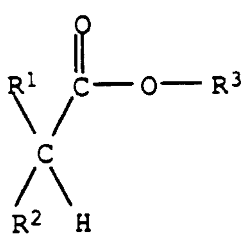

I

where

| | |
|---|---|
| $R^1$ and $R^2$ | are each hydrogen, $C_1$-$C_{12}$-alkyl, $C_3$-$C_8$-cycloalkyl, acyl, aryl or $C_7$-$C_{20}$-aralkyl or together $-(CH_2)_n-X-(CH_2)_m-$, |
| X | is methylene, oxygen, sulfur, NH or $NR^3$, |
| $R^3$ | is $C_1$-$C_{12}$-alkyl, and |
| n and m | are each from 0 to 8, |

which comprises reacting geminal dicarboxylic esters of the general formula II

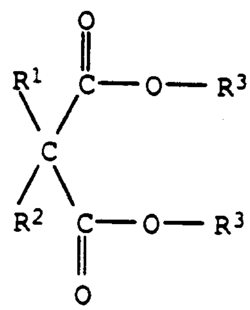

II

where $R^1$ to $R^3$ are each as defined above, at from 150 to 400 °C in the presence of acidic catalysts.

2. A process as claimed in claim 1, wherein the acidic catalysts used are metal oxides of the elements of main groups I to V and/or of subgroups 1 to 8 and/or of the lanthanide group of the periodic table of the elements and/or zeolites and/or heteropoly acids.

3. A process as claimed in claim 1, wherein the acidic catalysts used are aluminum oxide, silicon dioxide, titanium dioxide, zirconium dioxide, vanadium pentoxide, boron trioxide and/or oxides of chromium, molybdenum and/or tungsten.

4. A process as claimed in claim 1, wherein the catalysts used are zeolites or heteropoly acids.

5. A process as claimed in claim 1, wherein the reaction is carried out in the gas phase.

6. A process as claimed in claim 1, wherein $R^1$ and $R^2$ are together $-(CH_2)_n-X-(CH_2)_m-$, X is methylene, oxygen, sulfur, NH or $NR^3$, $R^3$ is $C_1-C_{12}$-alkyl, and n and m are each from 0 to 8 in the formulae I and II.

7. A process as claimed in claim 1, wherein $R^1$ and $R^2$ are together $-(CH_2)_2-O-(CH_2)_2-$ in the formulae I and II.

**Revendications**

1. Procédé de préparation d'esters d'acides monocarboxyliques de la formule générale I

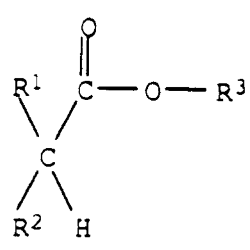

I,

dans laquelle

| | |
|---|---|
| $R^1$, $R^2$ | représentent chacun un atome d'hydrogène, un radical alkyle en $C_1$ à $C_{12}$, cycloalkyle en $C_3$ à $C_8$, acyle, aryle, ou aralkyle en $C_7$ à $C_{20}$, ou forment ensemble un radical de la formule $-(CH_2)_n-X-(CH_2)_m-$, |
| X | représente un radical méthylène, un atome d'oxygène, un atome de soufre, le radical NH ou $NR^3$, |
| $R^3$ | représente un groupe alkyle en $C_1$ à $C_{12}$ et |
| n, m | ont chacun une valeur qui varie de 0 à 8, |

caractérisé en ce que l'on fait réagir des esters d'acides dicarboxyliques géminaux de la formule générale II

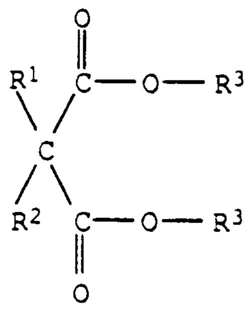

II,

dans laquelle les symboles $R^1$ à $R^3$ possèdent les significations qui leur ont été attribuées ci-dessus, en présence de catalyseurs acides et à des températures de 150 à 400 °C.

**2.** Procédé suivant la revendication 1, caractérisé en ce que, à titre de catalyseurs acides, on utilise des oxydes de métaux des éléments des premier à cinquième groupes principaux et/ou des premier à huitième sous-groupes et/ou du groupe des lanthanides du système périodique des éléments et/ou des zéolites et/ou des éthéropolyacides.

**3.** Procédé suivant la revendication 1, caractérisé en ce que, à titre de catalyseurs acides, on utilise de l'oxyde d'aluminium, du dioxyde de silicium, du dioxyde de titane, du dioxyde de zirconium, du pentoxyde de vanadium, du trioxyde de bore et/ou des oxydes du chrome, du molybdène et/ou du tungstène.

**4.** Procédé suivant la revendication 1, caractérisé en ce que, à titre de catalyseurs, on utilise des zéolites ou des hétéropolyacides.

**5.** Procédé suivant la revendication 1, caractérisé en ce que l'on travaille en phase gazeuse.

**6.** Procédé suivant la revendication 1, caractérisé en ce que $R^1$ et $R^2$ représentent ensemble un radical $-(CH_2)_n-X-(CH_2)_m-$, X représente un groupe méthylène, un atome d'oxygène, un atome de soufre, un radical NH ou $NR^3$, $R^3$ représente un radical alkyle en $C_1$ à $C_{12}$ et n et m ont des valeurs qui varient de 0 à 8 dans les formules I et II.

**7.** Procédé suivant la revendication 1, caractérisé en ce que $R^1$ et $R^2$ représentent ensemble le radical $-(CH_2)_2-O-(CH_2)_2-$ dans les formules I et II.